(19) Europäisches Patentamt
European Patent Office
Office européen des brevets

(11) Publication number: **0 438 848 A1**

(12) # EUROPEAN PATENT APPLICATION

(21) Application number: **90300807.6**

(22) Date of filing: **25.01.90**

(51) Int. Cl.5: **C10M 133/56**, //C10N30:04, C10N40:00,C10N60:00

(43) Date of publication of application: **31.07.91 Bulletin 91/31**

(84) Designated Contracting States: **AT BE CH DE DK ES FR GB IT LI LU NL SE**

(71) Applicant: **ETHYL PETROLEUM ADDITIVES LIMITED**
**London Road**
**Bracknell Berkshire, RG12 2UW(GB)**

(72) Inventor: **Scattergood, Roger**
**10 Grovelands Road Spencers Wood**
**Reading Berkshire RG7 1DP(GB)**
Inventor: **Walters, David Kenvyn 7 Firglen Drive**
**Yateley Camberley**
**Surrey GU17 7TR(GB)**

(74) Representative: **Collier, Jeremy Austin Grey et al**
**J.A.Kemp & Co. 14, South Square Gray's Inn**
**London WC1R 5EU(GB)**

(54) **Inhibiting fluoroelastomer degradation during lubrication.**

(57) A method of lubricating mechanical parts in the presence of at least one fluoroelastomer surface. The lubrication is effected by means of a lubricating oil containing a dispersant prepared by (i) reacting at least one polyamine with at least one acyclic hydrocarbyl substituted succinic acylating agent in which such acyclic hydrocarbyl substituent contains an average of at least 40 carbon atoms, such reaction being conducted using proportions such that the acylating agent is reacted with the polyamine in a mole ratio of from 1.05 to 2.85 moles per mole of polyamine, and (ii) reacting the product so formed with (a) at least one aliphatic vicinal dicarboxylic acid acylating agent containing 4 to 30 carbon atoms in the molecule and in which the two carboxyl groups are separated from each other by two aliphatic carbon atoms, or (b) an anhydride, acid halide, or ester of at least one such dicarboxylic acid acylating agent, or (c) a combination of (a) and (b), using in the reaction of (ii) proportions such that the mole ratio of such acylating agent is from 0.10 to 2.50 moles per mole of said polyamine with the proviso that the total mole ratio of the acylating agents in (i) and (ii) per mole of said polyamine is in the range of 2.40 to 4.50.

EP 0 438 848 A1

# INHIBITING FLUOROELASTOMER DEGRADATION DURING LUBRICATION

A continuing problem in the art of lubrication of mechanical systems such as internal combustion engines, gears, vehicular transmissions, hydraulic systems, and the like is to provide lubricant compositions which satisfy the demands imposed upon them by the original equipment manufacturers. One such requirement is that the lubricant not contribute to premature deterioration of seals, clutch plate facings or other fluoroelastomer parts which come in contact with the lubricant during service. Unfortunately, and as is well known, succinimide dispersants commonly used in lubricating oils and functional fluids tend to exhibit a strong adverse effect upon fluoroelastomers by causing them to lose their flexibility and strength properties, to become embrittled, and in severe cases, to disintegrate. It has been postulated that the co-presence of zinc-containing additives such as zinc dialkyldithiophosphates tends to increase the severity of this problem. A new practical cost-effective way of overcoming this serious problem would be a welcome contribution to the art.

In accordance with this invention, it has been discovered, inter alia, that succinimide dispersants formed in a particular manner from particular reactants exhibit significantly reduced tendencies of causing premature degradation of fluoroelastomers. Indeed, pursuant to preferred embodiments of this invention, fluoroelastomer degradation can be so effectively inhibited as to enable the lubricants and functional fluids provided by this invention to pass current test methods which must be passed in order for the lubricant to qualify for use in vehicles produced by certain original equipment manufacturers. And at the same time the effectiveness of the dispersants as dispersants is not materially impaired. Thus this invention provides new, practical, cost-effective ways of overcoming the problem referred to at the outset.

In one of its embodiments, this invention provides improved methods of lubricating mechanical parts in the presence of at least one fluoroelastomer surface, wherein the lubrication is effected by means of a lubricating oil or functional fluid containing a dispersant. The improvement involves providing as the lubricant for such lubrication, a lubricating oil or functional fluid containing a dispersant prepared by a process which comprises (i) reacting at least one polyamine with at least one oil soluble acyclic hydrocarbyl substituted succinic acylating agent in which such acyclic hydrocarbyl substituent contains an average of at least 40 carbon atoms, such reaction being conducted using proportions such that the acylating agent is reacted with the polyamine in a mole ratio of from 1.05 to 2.85 moles per mole of polyamine, and (ii) reacting the product so formed with (a) at least one aliphatic vicinal dicarboxylic acid acylating agent containing 4 to 30 carbon atoms in the molecule and in which the two carboxyl groups are separated from each other by two aliphatic carbon atoms, or (b) an anhydride, acid halide, or ester of at least one such dicarboxylic acid acylating agent, or (c) a combination of (a) and (b), using in the reaction of (ii) proportions such that the mole ratio of such acylating agent is from 0.10 to 2.50 moles per mole of said polyamine with the proviso that the total mole ratio of the acylating agents in (i) and (ii) per mole of said polyamine is in the range of 2.40 to 4.50.

In one preferred embodiment the mole ratio of acylating agent to polyamine in step (i) is at least 1.70 : 1.

In another preferred embodiment the proportions of the reactants are controlled such that the molar ratio of acylating agent(s) in (i) : acylating agent(s) in (ii) is above 1 : 1, and most preferably is at least 1.4 : 1.

In a particularly preferred embodiment the reactants are proportioned such that the total mole ratio of the acylating agents in (i) and (ii) per mole of the polyamine is in the range of 3.05 to 4.50.

Pursuant to still further embodiments of this invention, the compositions utilized pursuant to this invention additionally contain a zinc-containing additive complement, especially one or a mixture of zinc dihydrocarbyldithiophosphates, such as one or a combination of zinc dialkyldithiophosphates, one or a combination of zinc diaryldithiophosphates, or a combination of one or more zinc dialkyldithiophosphates with one or more zinc diaryldithiophosphates.

A feature of this invention is that the practice of the above methods results in a lower -- oftentimes a substantially lower -- amount of degradation of the fluoroelastomer contacted by the lubricating oil or functional fluid containing such dispersants as compared to the amount of degradation that would occur under the same conditions using the same oil or fluid composition containing the same quantity of succinimide dispersant made in the same way except for the exclusion of step (ii) above.

In another of its forms this invention provides in combination, (a) a mechanical mechanism containing moving parts to be lubricated, (b) a lubricating oil or functional fluid composition for lubricating such parts, and (c) a fluoroelastomer in contact with at least a portion of such lubricating oil or functional fluid during operation of such mechanism, characterized in that the lubricating oil or functional fluid composition for

effecting such lubrication contains as a dispersant therefor, a dispersant prepared by a process which comprises (i) reacting at least one polyamine with at least one oil soluble acyclic hydrocarbyl substituted succinic acylating agent in which such acyclic hydrocarbyl substituent contains an average of at least 40 carbon atoms, such reaction being conducted using proportions such that the acylating agent is reacted with the polyamine in a mole ratio of from 1.05 to 2.85 moles per mole of polyamine, and (ii) reacting the product so formed with (a) at least one aliphatic vicinal dicarboxylic acid acylating agent containing 4 to 30 carbon atoms in the molecule and in which the two carboxyl groups are separated from each other by two aliphatic carbon atoms, or (b) an anhydride, acid halide, or ester of at least one such dicarboxylic acid acylating agent, or (c) a combination of (a) and (b), using in the reaction of (ii) proportions such that the mole ratio of such acylating agent is from 0.10 to 2.50 moles per mole of said polyamine with the proviso that the total mole ratio of the acylating agents in (i) and (ii) per mole of said polyamine is in the range of 2.40 to 4.50. Among the mechanical mechanisms lubricated in this matter are the crankcases of internal combustion engines, gear boxes, vehicular transmissions, and hydraulic systems.

The above and still other embodiments and features of this invention will become still further apparent from the ensuing description and appended claims.

Acyclic hydrocarbyl-substituted succinic acid acylating agents and methods for their preparation are well known to those skilled in the art and are extensively reported in the patent literature. See, for example the following U.S. Patents:

| | | |
|---|---|---|
| 3,018,247 | 3,231,587 | 3,399,141 |
| 3,018,250 | 3,272,746 | 3,401,118 |
| 3,018,291 | 3,287,271 | 3,513,093 |
| 3,172,892 | 3,311,558 | 3,576,743 |
| 3,184,474 | 3,331,776 | 3,578,422 |
| 3,185,704 | 3,341,542 | 3,658,494 |
| 3,194,812 | 3,346,354 | 3,658,495 |
| 3,194,814 | 3,347,645 | 3,912,764 |
| 3,202,678 | 3,361,673 | 4,110,349 |
| 3,215,707 | 3,373,111 | 4,234,435 |
| 3,219,666 | 3,381,022 | |

As indicated in such prior patents, the acyclic hydrocarbyl substituted succinic acylating agents include the hydrocarbyl-substituted succinic acids, the hydrocarbyl-substituted succinic anhydrides, the hydrocarbyl-substituted succinic acid halides (especially the acid fluorides and acid chlorides), and the esters of the hydrocarbyl-substituted succinic acids and lower alcohols (e.g., those containing up to about 7 carbon atoms), that is, hydrocarbyl-substituted compounds which can function as carboxylic acylating agents. Of these compounds, the hydrocarbyl-substituted succinic acids and the hydrocarbyl-substituted succinic anhydrides and mixtures of such acids and anhydrides are generally preferred, the hydrocarbyl-substituted succinic anhydrides being particularly preferred.

The acyclic hydrocarbyl substituent on the succinic acid or acid derivative thereof used in step (i) of the present invention is preferably an alkyl or more preferably an alkenyl substituent having an average of at least about 40 carbon atoms, and preferably an average of at least about 64 carbon atoms. Preferred acylating agents for use in (i) have alkenyl substituents with a number average molecular weight (as determined by gel permeation chromatography) in the range of 980 to 5,000, most preferably in the range of 1,200 to 5,000, especially where the alkenyl substituents are formed from polyolefins made from $C_3$ or $C_4$ olefins (e.g., isobutylene, 1-butene, and mixtures of butenes containing the same as the predominant components), or combinations of such $C_3$ and $C_4$ olefins, or are formed from chlorinated polyolefins made from such $C_3$ or $C_4$ olefins or combinations thereof. Polyisobutenylsuccinic acids, polyisobutenylsuccinic anhydrides, and mixtures of polyisobutenylsuccinic acids and polyisobutenylsuccinic anhydrides are most especially preferred for use in the practice of step (i) above.

Suitable polyamines for use in step (i) above are described in many of the above cited U.S. Patents. For best results, the polyamines should contain at least two primary amino groups in the molecule.

The preferred polyamines used in the practice of this invention are the alkylene polyamines represented by the formula

$$H_2N(CH_2)_n(NH(CH_2)_n)_mNH_2$$

wherein each n is, independently, 2, 3, or 4 (preferably 2 to 3, and most preferably 2) and m is 0 to 10, (preferably 1 to 6). Illustrative are ethylene diamine, diethylene triamine, triethylene tetraamine, tetraethylene pentamine, pentaethylene hexamine, propylene diamine (1,3-propanediamine), butylene diamine (1,4-butanediamine), hexamethylene diamine (1,6-hexanediamine), decamethylene diamine (1,10-decanediamine), and the like. Preferred for use is tetraethylene pentamine or a mixture of ethylene polyamines which approximates tetraethylene pentamine such as "DOW E-100" (a commercial mixture available from Dow Chemical Company, Midland, Michigan).

As used herein the term "succinimide" is meant to encompass the completed reaction product from steps (i) and (ii) and is intended to encompass compounds wherein the product may have amide, amidine, and/or salt linkages in addition to the imide linkage of the type that results from the reaction of a primary amino group and an anhydride moiety.

The acylating agent utilized in step (ii) above is (1) one or a mixture of vicinal dicarboxylic acids containing from 4 to 30 carbon atoms in the molecule and characterized in that the two carboxyl groups are separated from each other by two aliphatic carbon atoms, or (2) one or a mixture of anhydrides, acid halides, or esters of such dicarboxylic acids, or (3) any combination of (1) and (2). Among suitable acylating agents for use in step (ii) are:

(a) one or a mixture of vicinal dicarboxylic acids of the formula

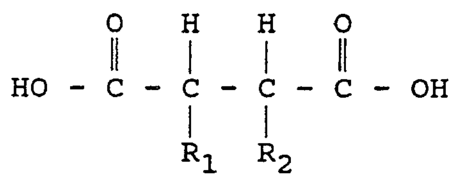

wherein each of $R_1$ and $R_2$ is, independently, a hydrogen atom, an alkyl or alkenyl group, or a hydroxyl group; or

(b) the anhydride, acid halide, or ester of such vicinal dicarboxylic acid(s) of (a); or

(c) a combination of at least one component from (a) and at least one component from (b); or

(d) one or a mixture of vicinal dicarboxylic acids of the formula

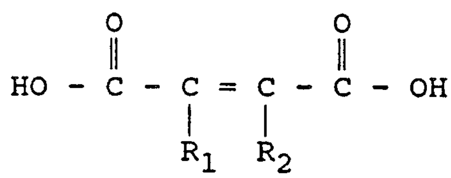

wherein each of $R_1$ and $R_2$ is, independently, a hydrogen atom, or an alkyl or alkenyl group; or

(e) the anhydride, acid halide, or ester of such vicinal dicarboxylic acid(s) of (d) or;

(f) a combination of at least one component from (d) and at least one component from (e); or

(g) any combination of at least one component from (a), (b), and/or (c), and at least one component from (d), (e), and/or (f).

Such acylating agent thus encompasses such compounds as maleic anhydride, maleic acid, fumaric acid, malic acid, thiomalic acid, tartaric acid, itaconic acid, itaconic anhydride, citraconic acid, citraconic anhydride, mesaconic acid, chloromaleic acid, ethylmaleic anhydride, dimethylmaleic anhydride, ethylmaleic acid, dimethylmaleic acid, hexylmaleic acid, succinic acid, succinic anhydride, butylsuccinic anhydride, octenylsuccinic anhydride, hexenylsuccinic anhydride, octadecenylsuccinic anhydride, eicosenylsuccinic anhydride, docosenylsuccinic anhydride and the corresponding acid halides, or esters which are preferably esters of lower alcohols.

As noted above, the succinimide dispersants utilized pursuant to this invention are prepared by a process which comprises (i) reacting at least one polyamine with at least one acyclic hydrocarbyl substituted succinic acylating agent in which such acyclic hydrocarbyl substituent contains an average of at least 40 carbon atoms, such reaction being conducted using proportions such that the acylating agent is reacted with the polyamine in a mole ratio of from 1.05 to 2.85 moles per mole of polyamine, and (ii) reacting the product so formed with (a) at least one aliphatic vicinal dicarboxylic acid acylating agent containing 4 to 30 carbon atoms in the molecule and in which the two carboxyl groups are separated from

each other by two aliphatic carbon atoms, or (b) an anhydride, acid halide, or ester of at least one such dicarboxylic acid acylating agent, or (c) a combination of (a) and (b), using in the reaction of (ii) proportions such that the mole ratio of such acylating agent is from 0.10 to 2.50 moles per mole of said polyamine with the proviso that the total mole ratio of the acylating agents in (i) and (ii) per mole of said polyamine is in the range of 2.40 to 4.50, and preferably in the range of 3.05 to 4.50.

The reactions involved in steps (i) and (ii) are conducted at conventional temperatures in the range of 80°C to 200°C, more preferably 140°C to 180°C. These reactions may be conducted in the presence or absence of an ancillary diluent or liquid reaction medium, such as a mineral lubricating oil solvent. If the reaction is conducted in the absence of an ancillary solvent of this type, such is usually added to the reaction product on completion of the reaction. In this way, the final product is in the form of a convenient solution in lubricating oil and thus is compatible with a lubricating oil base stock. Suitable solvent oils are the same as the oils used as a lubricating oil base stock and these generally include lubricating oils having a viscosity (ASTM D 445) of 2 to 40, preferably 3 to 12 $mm^2/sec$ at 100°C, with the primarily paraffinic mineral oils such as Solvent 100 Neutral being particularly preferred. Other types of lubricating oil base stocks can be used, such as synthetic lubricants including polyesters, poly-$\alpha$-olefins (e.g., hydrogenated or unhydrogenated $\alpha$-olefin oligomers such as hydrogenated poly-1-decene), and the like. Blends of mineral oil and synthetic lubricating oils are also suitable for various applications in accordance with this invention.

Finished lubricating oil compositions of this invention are prepared containing the dispersant of this invention together with conventional amounts of other additives to provide their normal attendant functions. Thus use may be made of such conventional additives as viscosity index improvers, dispersant viscosity index improvers, rust inhibitors, metal detergent additives, antioxidants, antiwear additives, extreme pressure additives, and the like. Reference may be had to the various U.S. patents referred to hereinabove for exemplary disclosures of various conventionally used additives for lubricating oils.

A particularly preferred ancillary additive used in the lubricant compositions is a grafted copolymer dispersant VI improver of the type described in U.S. Pat. No. 4,519,929.

Preferred lubricants and functional fluids provided for use and used pursuant to this invention include those which pass either (a) the Volkswagen P.VW 3334 Seal Test, or (b) the CCMC Viton Seal Test, CEC L-39-T-87 Oil/Elastomer Compatibility Test, or most preferably, both such tests. The Volkswagen P.VW 3334 Seal Test involves keeping a test specimen of fluoroelastomer (VITON AK6) in an oil blend at 150°C for 96 hours and then comparing both the change in elongation to break and the tensile strength of the test specimen to the corresponding properties of a fresh specimen of the same fluoroelastomer. The exposed test specimen is also examined for the presence of cracks. In these tests, a lubricant passes the test if the exposed test specimen exhibits a change in elongation to break (as compared to an untested specimen) of no more than -25% and a tensile strength (as compared to an untested specimen) of no more than -20%, and possesses no cracks. The CCMC Viton Seal Test is similar to the VW Test except that it is a 7-day test rather than a 4-day test, the elastomer is VITON RE I, and the pass/fail points are -50% tensile strength and -60% elongation.

The practice and the benefits achievable by the practice of this invention are illustrated in the following specific examples which are not to be construed as limitations on this invention.

EXAMPLE 1

In a first stage reaction, polyisobutenylsuccinic anhydride (PIBSA) formed from polyisobutylene (number average molecular weight = 1300) and tetraethylene pentamine (TEPA) in a mole ratio of 1.8:1 are reacted at 165-170°C for 4 hours. In a second stage reaction, maleic anhydride (MA) is added to the first stage reaction product in amount equivalent to 1.25 moles per mole of TEPA used in the first stage and the resultant mixture is heated at 165-170°C for 1.5 hours. The succinimide is thus formed using a total mole ratio of anhydrides to TEPA of 3.05:1. The mole ratio of PIBSA:MA in this synthesis is 1.44:1. To provide a handleable concentrate, the reaction product is suitably diluted with 100 solvent neutral mineral oil such that the nitrogen content of the blend is about 1.8%.

EXAMPLE 2

The procedure of Example 1 is repeated except that in the first stage the PIBSA and TEPA are reacted in a mole ratio of 2.05:1 and in the second stage the MA is used in amount equivalent to a mole ratio of 1:1 relative to the TEPA used in the first stage. Thus the total mole ratio of anhydrides to polyamine is again 3.05:1. The mole ratio of PIBSA:MA in this synthesis is 2.05:1.

EXAMPLE 3

The same general procedure as in Example 1 is employed except that the PIBSA:TEPA mole ratio in the first stage is 2.3:1 and that in the second stage the MA is used in amount equivalent to a mole ratio of 1:1 relative to the TEPA used in the first stage. The total mole ratio of anhydrides to polyamine is thus 3.3:1, and the mole ratio of PIBSA:MA is 2.3:1.

EXAMPLE 4

The procedure of Example 1 is repeated except that in the first stage the PIBSA and TEPA are reacted in a mole ratio of 2.05:1 and in the second stage the MA is used in amount equivalent to a mole ratio of 2:1 relative to the TEPA used in the first stage. Thus the total mole ratio of anhydrides to polyamine is 4.05:1, and the PIBSA:MA mole ratio is 1.03:1.

EXAMPLE 5

The procedure of Example 3 is repeated except that in the first stage the PIBSA:TEPA mole ratio is 1.5:1. Thus the total mole ratio of acylating agents to polyamine is 2.5:1.

EXAMPLE 6

In a first stage reaction, PIBSA formed from polyisobutylene (number average molecular weight = 1300) and TEPA in a mole ratio of 2.05:1 are reacted at 165-170 °C for 4 hours. In a second stage reaction, maleic acid is added to the first stage reaction product in amount equivalent to one mole per mole of TEPA used in the first stage and the resultant mixture is heated at 165-170 °C for 1.5 hours. The succinimide is thus formed using a total mole ratio of acylating agents to TEPA of 3.05:1, and the mole ratio of PIBSA to maleic acid is 2.05:1. As in Example 1, the reaction product is suitably diluted with mineral oil base stock to provide a handleable concentrate.

EXAMPLE 7

The procedure of Example 6 is repeated except that fumaric acid is used in the second stage in amount equivalent to a mole ratio of 1:1 relative to the TEPA used in the first stage. Thus the total mole ratio of acylating agents to polyamine is 3.05:1, and the mole ratio of PIBSA to fumaric acid is 2.05:1.

EXAMPLE 8

The procedure of Example 7 is repeated using an equivalent amount of malic acid in lieu of fumaric acid in the second stage. The total mole ratio of acylating agents to polyamine is 3.05:1, and the mole ratio of PIBSA to malic acid is 2.05:1.

EXAMPLE 9

The procedure of Example 8 is repeated using in the second stage an equivalent amount of succinic acid in lieu of malic acid. The total mole ratio of of acylating agents to polyamine is 3.05:1, and the mole ratio of PIBSA to succinic acid is 2.05:1.

EXAMPLE 10

Example 9 is repeated except that 2 moles of succinic acid per mole of TEPA are employed in the second stage such that the total mole ratio of acylating agents to polyamine is 4.05:1, and the mole ratio of PIBSA to succinic acid is 1.03:1.

EXAMPLE 11

In the first stage, PIBSA formed from polyisobutylene (number average molecular weight = 1300) and TEPA in a mole ratio of 2.3:1 are reacted at 165-170 °C for 4 hours. In the second stage, succinic anhydride is added to the first stage reaction product in amount equivalent to one mole per mole of TEPA used in the

first stage and the resultant mixture is heated at 165-170°C for 1.5 hours. The succinimide is thus formed using a total mole ratio of acylating agents to TEPA of 3.3;1, and the mole ratio of PIBSA to succinic anhydride is 2.3:1.

EXAMPLE 12

The first stage reaction involves reaction of PIBSA and TEPA in a mole ratio of 2.05:1. The reaction is conducted at 165-170°C for 4 hours. In the second stage, an alkenyl succinic anhydride in which the alkenyl group contains an average of between 20 and 24 carbon atoms is employed in an amount equivalent to one mole per mole of TEPA used in the first stage, and the reaction is conducted at 165-170°C for 1.5 hours. The resultant succinimide is thus formed using a total mole ratio of acylating agents to TEPA of 3.05:1. The mole ratio of PIBSA to alkenyl succinic anhydride is 2.05:1. For ease of handling, the result product is diluted with mineral oil.

EXAMPLE 13

Using the procedure of Example 12, PIBSA is reacted with TEPA in the first stage in a mole ratio of 2.3:1. In the second stage, maleic anhydride is reacted using 0.75 mole per mole of TEPA used in the first stage. The product thus is formed with a total mole ratio of acylating agents to TEPA of 3.05:1. The mole ratio of PIBSA to MA is 3.07:1.

EXAMPLE 14

The procedure of Example 13 is repeated substituting an equivalent amount of fumaric acid for the maleic anhydride in the second stage. The total mole ratio of acylating agent to TEPA is thus 3.05:1. The mole ratio of PIBSA to fumaric acid is 3.07.1.

COMPARATIVE EXAMPLES A - H

A group of succinimides was produced in which the PIBSA:TEPA mole ratio in the first stage was 2.05:1. In the respective second stages, individual portions of the resultant product were reacted individually with oxalic acid, adipic acid, octanoic acid, acetic acid, formic acid or phthalic anhydride. The makeup of these succinimides is as follows:

| Comparative Example | Acid | Total Mole Ratio of PIBSA + Acid:TEPA |
|---|---|---|
| A | Oxalic | 3.05:1 |
| B | Adipic | 3.05:1 |
| C | Octanoic | 3.05:1 |
| D | Acetic | 4.05:1 |
| E | Formic | 4.05:1 |
| F | Octanoic | 4.05:1 |
| G | Phthalic* | 3.05:1 |
| H | Acetic | 3.05:1 |

* Used in the form of phthalic anhydride

Finished crankcase lubricating oils for use in internal combustion engines containing various substituted

succinimide dispersants were formulated. Except for Test No. 3 wherein a diesel engine crankcase formulation was employed, each such oil contained, in addition to the succinimide dispersant, conventional amounts of overbased sulfonates, zinc dialkyl dithiophosphate, antioxidant, viscosity index improver, rust inhibitor, and antifoam agent to provide an SAE 15W/40 crankcase lubricant oil. Each such lubricant contained an amount of the succinimide dispersant to provide a nitrogen content of 0.13%.

The resultant finished lubricating oils were subjected to the Volkswagen P.VW 3334 Seal Test. The results of this series of tests are summarized in Table 1.

Table 1 - Results of Volkswagen Seal Tests

| Test No. | Succinimide Used | Change in Elongation to Break Compared to Fresh Seal, % | Tensile Strength Compared to Fresh Seal, % | Cracking |
|---|---|---|---|---|
| 1 | Example 1 | -6 | -15 | Pass |
| 2 | Example 2 | -9 | -18 | Pass |
| 3 | Example 2 | None | +4 | Pass |
| 4 | Example 3 | +3 | -2 | Pass |
| 5 | Example 4 | +2 | -11 | Pass |
| 6 | Example 5 | -29 | -35 | Pass |
| 7 | Example 6 | -10 | -10 | Pass |
| 8 | Example 7 | -17 | -17 | Pass |
| 9 | Example 8 | -13 | -14 | Pass |
| 10 | Example 9 | -33 | -36 | Pass |
| 11 | Example 10 | -19 | -17 | Pass |
| 12 | Example 11 | -27 | -28 | Pass |

| | | | | |
|---|---|---|---|---|
| 13 | Example 12 | -30 | -33 | Fail |
| 14 | Control* | -45 | -58 | Fail |
| 15 | Example A | -41 | -46 | Fail |
| 16 | Example B | -39 | -44 | Fail |
| 17 | Example C | -59 | -59 | Fail |
| 18 | Example D | -48 | -55 | Fail |
| 19 | Example E | -46 | -51 | Fail |
| 20 | Example F | -60 | -61 | Fail |
| 21 | Example G | -45 | -52 | Fail |

* Formulation containing commercial succinimide dispersant

It will be noted that in all of the tests conducted pursuant to this invention (Test nos. 1-13) less degradation of the fluoroelastomer occurred as compared to the degradation which occurred when using a widely used commercially available crankcase lubricating oil formulation containing a commercial succinimide dispersant which had not been subjected to second stage treatment. In fact, all of the runs pursuant to this invention passed the Volkswagen Test with the exception of the products of Examples 5, 9, 11, and 12 (Tests 6, 10, 12, and 13). It is also interesting to note that in Tests 15-21 the amount of fluoroelastomer degradation was in general equal to or worse than the degradation that occurred when using the commercial succinimide dispersant.

Another series of tests was conducted using the CCMC Seal Test procedure. The results of these tests are summarized in Table 2.

Table 2 - Results of CCMC Seal Tests

| Test No. | Succinimide Used | Change in Elongation to Break Compared to Fresh Seal, % | Tensile Strength Compared to Fresh Seal, % |
|---|---|---|---|
| 1 | Example 9 | -36.3 | -3.4 |
| 2 | Example 12 | -32.5 | -1.8 |
| 3 | Control* | -50.0 | -31.2 |
| 4 | Example B | -42.3 | -12.8 |
| 5 | Example G | -49.1 | -26.2 |
| 6 | Example H | -58.5 | -38.9 |

* Formulation containing commercial succinimide dispersant

The data in Table 2 indicate that the test conditions of the CCMC Test are substantially less severe

than those of the Volkswagen Test. They also indicate that the degradation which occurred in Tests 1 and 2 pursuant to this invention was less than in the runs not conducted pursuant to this invention (Tests 3-6).

The dispersants utilized according to the invention can be incorporated in a wide variety of lubricants. They can be used in lubricating oil compositions, such as automotive crankcase lubricating oils, automatic transmission fluids and gear oils in effective amounts to provide active ingredient concentrations in finished formulations generally within the range of 0.5 to 10 weight percent, for example, 1 to 9 weight percent, preferably 2 to 8 weight percent, of the total composition. Conventionally, the dispersants are admixed with the lubricating oils as dispersant solution concentrates which usually contain up to about 50 weight percent of the active ingredient additive compound dissolved in mineral oil, preferably a mineral oil having an ASTM D-445 viscosity of 2 to 40, preferably 3 to 12 centistokes at 100°C. The lubricating oil not only can be hydrocarbon oils of lubricating viscosity derived from petroleum but also can be natural oils of suitable viscosities such as rapeseed oil, and synthetic lubricating oils such as hydrogenated polyolefin oils; poly-$\alpha$-olefins (e.g., hydrogenated or unhydrogenated $\alpha$-olefin oligomers such as hydrogenated poly-1-decene); alkyl esters of dicarboxylic acids; complex esters of dicarboxylic acid, polyglycol and alcohol; alkyl esters of carbonic or phosphoric acids; polysilicones; fluorohydrocarbon oils; and mixtures or lubricating oils and synthetic oils in any proportion. The term "lubricating oil" for this disclosure includes all the foregoing. The useful dispersant may be conveniently dispersed as a concentrate of 10 to 80 weight percent of mineral oil, e.g., Solvent 100 Neutral oil with or without other additives being present and such concentrates are a further embodiment of this invention.

As noted above, such lubricating oils compositions containing the dispersants of the present invention will also contain other well-known additives such as the zinc dialkyl ($C_3$-$C_8$) and/or diaryl ($C_6$-$C_{10}$) dithiophosphate wear inhibitors, generally present in amounts of 0.5 to 5 weight percent. Useful detergents include the oil-soluble normal basic or overbased metal, e.g., calcium, magnesium, and barium, salts of petroleum naphthenic acids, petroleum sulfonic acids, alkyl benzene sulfonic acids, oil-soluble fatty acids, alkyl salicylic acids, sulfurized or unsulfurized alkyl phenates, and hydrolyzed or unhydrolyzed phosphosulfurized polyolefins. Gasoline engine crankcase lubricants typically contain, for example, from 0.5 to 5 weight percent of one or more detergent additives. Diesel engine crankcase oils may contain substantially higher level of detergent additives. Preferred detergents are the calcium and magnesium normal or overbased phenates, sulfurized phenates or sulfonates.

Oxidation inhibitors include hindered phenols (e.g., 2,6-di-tert-butyl-para-cresol, 2,6-di-tert-butylphenol, 4,4'-methylenebis(2,6-di-tert-butylphenol), and mixed methylene bridged polyalkyl phenols), amines, sulfurized phenols and alkyl phenothiazines. Antioxidants are usually present in the lubricant in amounts of from 0.001 to 1 weight percent.

Pour point depressants which may be present in the oil in amounts of from 0.01 to 1 weight percent include wax alkylated aromatic hydrocarbons, olefin polymers and copolymers, and acrylate and methacrylate polymers and copolymers.

Viscosity index improvers, the concentrations of which may in the lubricants vary from 0.2 to 15 weight percent, (preferably from 0.5 to 5 weight percent) depending on the viscosity grade required, include hydrocarbon polymers grafted with, for example, nitrogen-containing monomers, olefin polymers such as polybutene, ethylene-propylene copolymers, hydrogenated polymers and copolymers and terpolymers of styrene with isoprene and/or butadiene, polymers of alkyl acrylates or alkyl methacrylates, copolymers of alkyl methacrylates with N-vinyl pyrrolidone or dimethylaminoalkyl methacrylate, post-grafted polymers of ethylene-propylene with an active monomer such as maleic anhydride which may be further reacted with an alcohol or an alkylene polyamine, and styrene/maleic anhydride polymers post-treated with alcohols and amines.

Antiwear activity can be provided by 0.01 to 2 weight percent of the aforementioned metal dihydrocarbyl dithiophosphates and the corresponding precursor esters, phosphosulfurized pinenes, sulfurized olefins and hydrocarbons, sulfurized fatty esters and alkyl polysulfides. Preferred are the zinc dihydrocarbyl dithiophosphates which are salts of dihydrocarbyl esters of dithiophosphoric acids.

Other additives include effective amounts of friction modifiers or fuel economy additives such as the alkyl phosphonates as disclosed in U.S. 4,356,097, aliphatic hydrocarbyl substituted succinimides as disclosed in EPO 0020037, dimer acid esters, as disclosed in U.S. 4,105,571, oleimide which is present in the oil in amounts of 0.1 to 5 weight percent. Glycerol oleates are another example of fuel economy additives and these are usually present in very small amounts, such as 0.05 to 0.2 weight percent based on the weight of the formulated oil.

**Claims**

1. A method of lubricating mechanical parts with a lubricating oil containing a dispersant in the presence of at least one fluoroelastomer surface; characterized in that the lubrication is performed with a lubricating oil containing a dispersant prepared by a process which comprises (i) reacting at least one polyamine with at least one acyclic hydrocarbyl substituted succinic acylating agent in which such acyclic hydrocarbyl substituent contains an average of at least 40 carbon atoms, such reaction being conducted using proportions such that the acylating agent is reacted with the polyamine in a mole ratio of from 1.05 to 2.85 moles per mole of polyamine, and (ii) reacting the product so formed with (a) at least one aliphatic vicinal dicarboxylic acid acylating agent containing 4 to 30 carbon atoms in the molecule and in which the two carboxyl groups are separated from each other by two aliphatic carbon atoms, or (b) an anhydride, acid halide, or ester of at least one such dicarboxylic acid acylating agent, or (c) a combination of (a) and (b), using in the reaction of (ii) proportions such that the mole ratio of such acylating agent is from 0.10 to 2.50 moles per mole of said polyamine with the proviso that the total mole ratio of the acylating agents in (i) and (ii) per mole of said polyamine is in the range of 2.40 to 4.50.

2. A method of Claim 1 wherein the acyclic hydrocarbyl substituent of the acylating agent(s) employed in (i) is predominantly or entirely an alkyl or alkenyl substituent.

3. A method of Claim 1 or 2 wherein the polyamine is predominantly or entirely alkylene polyamine of the formula

$H_2N(CH_2)_n(NH(CH_2)_n)_mNH_2$

wherein each n is, independently, 2, 3 or 4 and m is 0 to 10.

4. A method of any of the preceding claims wherein the acylating agent(s) employed in (ii) comprises (a) one or a mixture of aliphatic vicinal dicarboxylic acids of the formula

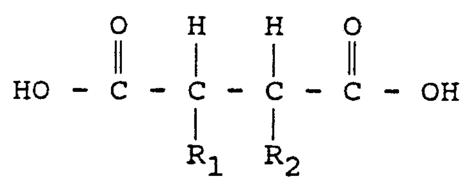

wherein each of $R_1$ and $R_2$ is, independently, a hydrogen atom, an alkyl or alkenyl group, or a hydroxyl group; or (b) the anhydride, acid halide, or ester of such vicinal dicarboxylic acid(s); or (c) a combination of at least one component from (a) and at least one component from (b); or

(d) one or a mixture of vicinal dicarboxylic acids of the formula

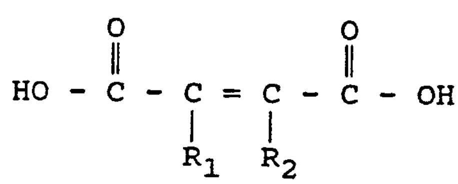

wherein each of $R_1$ and $R_2$ is, independently, a hydrogen atom, an alkyl or alkenyl group; or

(e) the anhydride, acid halide, or ester of such vicinal dicarboxylic acid(s) of (d) or;

(f) a combination of at least one component from (d) and at least one component from (e); or

(g) any combination of at least one component from (a), (b), and/or (c), and at least one component from (d), (e), and/or (f).

5. A method of any of the preceding claims wherein:

1) the acyclic hydrocarbyl substituted succinic acylating agent employed in (i) is predominantly or entirely polyisobutenyl succinic acid or polyisobutenyl succinic acid and polyisobutenyl succinic

anhydride in which the number average molecular weight of the isobutenyl substituents of such acylating agent falls in the range of 980 to 5,000; and

2) the polyamine is (a) tetraethylene pentamine or a combination of ethylene polyamines which approximate tetraethylene pentamine, or (b) triethylene tetraamine or a combination of ethylene polyamines which approximate triethylene tetraamine, or (c) a combination of at least one component of (a) and at least one component of (b).

6. A method of any of the preceding claims wherein the acylating agent employed in (ii) is predominantly or entirely maleic acid, fumaric acid, malic acid, maleic anhydride or any combination of two or more of the foregoing.

7. A method of any of the preceding claims wherein said total mole ratio is at least 3.05 moles of acylating agents per mole of polyamine.

8. A method of any of the preceding claims wherein the reactants are employed in relative proportions such that the molar ratio of acylating agent(s) in (i) : acylating agent(s) in (ii) is above 1:1.

9. A method of Claim 8 wherein the reactants are employed in relative proportions such that the molar ratio of acylating agent(s) in (i) : acylating agent(s) in (ii) is at least 1.4:1.

10. In combination, a mechanical mechanism containing moving parts to be lubricated, a lubricating oil composition for lubricating such parts, and a polyfluoroelastomer in contact with at least a portion of such lubricating oil composition, characterized in that the lubricating oil composition for effecting such lubrication contains as a dispersant therefor, a dispersant prepared by a process which comprises (i) reacting at least one polyamine with at least one acyclic hydrocarbyl substituted succinic acylating agent in which such acyclic hydrocarbyl substituent contains an average of at least 40 carbon atoms, such reaction being conducted using proportions such that the acylating agent is reacted with the polyamine in a mole ratio of from 1.05 to 2.85 moles per mole of polyamine, and (ii) reacting the product so formed with (a) at least one aliphatic vicinal dicarboxylic acid acylating agent containing 4 to 30 carbon atoms in the molecule and in which the two carboxyl groups are separated from each other by two aliphatic carbon atoms, or (b) an anhydride, acid halide, or ester of at least one such dicarboxylic acid acylating agent, or (c) a combination of (a) and (b), using in the reaction of (ii) proportions such that the mole ratio of such acylating agent is from 0.10 to 2.50 moles per mole of said polyamine with the proviso that the total mole ratio of the acylating agents in (i) and (ii) per mole of said polyamine is in the range of 2.40 to 4.50.

European Patent Office

# EUROPEAN SEARCH REPORT

Application Number

EP 90 30 0807

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | GB-A-2 140 811 (EXXON RES. AND ENG. CO.)<br>* Page 1, lines 27-42; page 2, lines 1-5; page 3, lines 13-25,55-60; page 4, lines 6-11 * | 1-6,8-10 | C 10 M 133/56 //<br>C 10 N 30:04<br>C 10 N 40:00<br>C 10 N 60:00 |
| X | EP-A-0 072 645 (EXXON RES. AND ENG. CO.)<br>* Page 9, line 24 - page 11, line 3 * | 1-6,8-10 | |
| A | US-A-4 663 064 (T.E. NALESNIK)<br>* Column 1, lines 45-60; claims 1-3; column 3, line 44 - column 5, line 15 * | 1-3,5,7-10 | |
| A | GB-A-1 018 982 (ESSO RES. AND ENG. CO.)<br>* Page 1, lines 24-36; page 2, lines 14-22; page 6, example II; claims 1,14-16; page 10, example VI * | 1-6,8-10 | |

TECHNICAL FIELDS SEARCHED (Int. Cl.5)

C 10 M
C 08 F

The present search report has been drawn up for all claims

| Place of search | Date of completion of the search | Examiner |
|---|---|---|
| THE HAGUE | 07-09-1990 | HILGENGA K.J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document